# EUROPEAN PATENT APPLICATION

(11) **EP 3 799 891 A1**
(43) Date of publication of application: **07.04.2021**
(21) Application number: 19201160.9
(22) Date of filing: 02.10.2019
(51) Int. Cl.: A61L 15/26, A61L 15/28, A61L 15/44, A61L 15/46, A61K 8/02, A61L 31/04, A61L 31/06, A61L 31/16, A61L 31/12

(54) **3D-PATTERNED FIBER MATERIAL FOR THE TOPICAL DELIVERY OF NUCLEIC ACID AND THE PROCESS FOR ITS PREPARATION**

(71) Applicant: Universita' Degli Studi Di Pavia, 27100 Pavia (IT)
(72) Inventor: DORATI, Rossella, I-27100 PAVIA (IT); CONTI, Bice, I-27100 PAVIA (IT); GENTA, Ida, I-27100 PAVIA (IT)
(74) Representative: Croce, Valeria

(57) **Abstract**

The present invention discloses a three-dimensional platform delivery technology comprising a polymeric material.

## Description

### Field of the invention

The present invention pertains the medical field and, in particular, it relates to a platform delivery technology of nucleic acids for preventing hypertrophic scars and treating burn wounds and the like.

### Background of the invention

Burn injury is a serious damage of skin and other tissues leading to severe morbidity and significant mortality, and considerable health-economic impact. Depending on the wounding mechanism, the scarring wound healing response has a pathological spectrum, ranging from cosmetic annoyance to grave functional impairment (i.e. scar traction across joints, impeding facial muscular movement). In particular, severe burn involving face and upper body generally leads to disfigurement, scar contractures and hypertrophic scars.

Approaches to optimize healing potential of burn wounds are targeted wound care, and where appropriate surgery to minimize the development of hypertrophic scarring. Such approaches often fail, and modulation of the established scar is continued although the optimal indication, timing, and combination of therapies have yet to be established. The need for novel treatments is paramount, and future efforts to improve outcomes and quality of life should include optimization of wound healing to attenuate or prevent hypertrophic scarring, with millions of people annually suffering from these discomforts. Since traditional management rarely provides satisfactory outcomes, it is crucial to develop an effective strategy to prevent hypertrophic scarring.

Current treatment strategies for extensive burns are limited to the use of autografts, allografts, and skin substitutes, and these often result in scarring, infection, and graft failure. The majority of skin substitutes available for clinical practice contain allogenic biological products, and the risk of disease transmission poses as a limitation particularly for natural biological skin substitutes. Despite extensive and strict sterilization procedures, current methods are insufficient to certify biological skin substitutes to be free from any unknown diseases or prion diseases from animal material. In contrast to biological substitutes, synthetic substitutes are free from any risk of disease transmission. However, only few synthetic skin substitutes are on the market today. Although synthetic materials have greater structural integrity compared to natural products, poor bioactivity remains a major concern. Moreover, poor host response may lead to negative effects on quality of scar.

The topical application of therapeutic agents has shown promising efficacy in the treatment of skin disorders, such as protective agents, anti-itch agents, anti-infective agents, anti-inflammatory agents, and nucleic acids. Therapeutics based on nucleic acids, which act by inhibiting the expression of target transcripts, represent a novel class of potent and highly specific next-generation treatments for human skin diseases. Topical delivery of nucleic acids represents an appealing site of application specifically to target a mutant gene locally and avoid the systemic exposure of nucleic acid to the whole body, which has consequences on the overall health and compliance regarding the off targeting and side effects.

Nucleic acids are primarily used in gene therapy field as a treatment showing a prominent effect in reducing the expression of a specific gene. Due to its high activity and precise gene selectivity, siRNA is expected to be an alternative treatment to antisense oligodeoxynucleotide (ODN) currently being used as a therapeutic agent as a result of the past 20-year's research. Accordingly, more than thirty pharmaceutical and biotechnology companies have concentrated on the development of a drug product based on siRNA. Particularly, the development of siRNA-related therapy for treating diabetes, obesity, rheumatism, Parkinson's disease, B/C-type hepatitis, AIDS, and cancer is in progress.

### siRNA

siRNA, which is a short, double-stranded RNA consisting of about 19 to 23 nucleotides, suppresses the expression of a gene by targeting the mRNA of a target gene having a complementary base sequence to them. In other words, a mRNA regulating the expression metabolic process of a specific gene is singularly degraded to stop the protein synthesis of the target gene, thereby treating a disease.

Accordingly, studies on delivery carriers using cationic liposomes or micelles and cationic polymers for delivering siRNA, having strong negative charge, into a living body, have been carried out. siRNA has a low stability and thus it is degraded in a short period of time by a variety of enzymes existing in plasma in large quantities *in vivo.* Particularly, in case of an injection treatment, siRNA is more quickly destroyed if not stably treated, and it hardly permeates membranes having negative charge due to its cationic property, and as a result, the transmissivity into cells is reduced, thereby causing a problem that the treatment efficiency is rapidly reduced. Although siRNA consists of double strands, the binding of ribose sugars constituting a RNA is very unstable chemically compared to the binding of deoxyribose sugars constituting a DNA, and thus most of them are rapidly degraded *in vivo* with a half-life of around 30 minutes. Furthermore, siRNA is recognized as foreign substance *in vivo,* thereby causing adverse effects on an immune system.

Moreover, siRNA affects other portions of the gene that are not an originally planned portion of the gene, thereby causing cross-hybridization in a gene base sequence.

A number of specialized primary wound dressings are currently available for treating partial-thickness burns (both superficial and deep partial-thickness burns) and other types of wounds, characterized by low to high levels of wound exudates. Such dressings are specifically designed to absorb the wound exudate, thus keeping the moist environment and facilitating debridement of necrotic tissue and spontaneous re-epithelialization of the skin, while providing temporary and prompt wound coverage and mechanical barrier to infections.

Several dressings might also include specific active agents (antibiotic or antimicrobial agents) in their formulation, in order to further protect the wound bed from undesired microbial contaminations.

### Electrospinning technology

The electrospinning technology is known for its ability of fabricating fibers. More than 100 different polymers have been successfully electrospun into ultrafine fibers using this technique including synthetic and natural polymers. Despite the promising characteristics of nanofibers, no products are already on the market for clinical biomedical application. The sterile product on the market based on nanofiber are NanoAligned™ or NanoECM™ Randomly Oriented Polymer Nanofibers multi-well plates used for *in vitro* studies, standard plates include polycaprolactone (PCL) nanofibers averaging less than one micron in diameter; moreover, the fiber dimensions and specific physical properties are optimized to produce ideal synthetic *in vitro* models.

These culture plates are sterilized after packaging (probably gamma ray sterilization) and may be used as direct replacements for polystyrene tissue culture in any traditional cell culture application.

The major issue related to electrospinning production, in particular for pharmaceutical and biotechnological applications, is the sterilization. Gamma ray treatment may be a viable solution for the sterilization of medical device based on nanofiber sensitive to steam and heat; however, the same treatment could not be considered the proper option for ATMPs products, whether they are engineered 3D scaffold for tissue engineered or gene therapy medicinal products.

### Summary of the invention

The authors of the present invention have surprisingly developed a fiber platform, preferably a three-dimensional fiber platform, which can be used for the controlled and sustained delivery of drug, for absorbing the wound exudate and for supporting and promoting cell attachment and proliferation.

### Object of the invention

In a first object, the present invention discloses a process for the preparation of a new material.

In a preferred object, the material can be in the form of a fiber delivery platform, a fiber dressing or a fiber scaffold.

In a second object, the present invention discloses a process for the preparation of a fiber delivery platform, a fiber dressing or a fiber scaffold loaded with a drug or an active agent.

In a preferred embodiment, the active agent is represented by siRNA.

In a third object, the present invention discloses a fiber delivery platform, a fiber dressing or a fiber scaffold, optionally loaded with a drug or an active agent, for use as a medicament.

Particularly, it discloses the use as a medicament for the treatment and healing of wounds.

In a preferred object, wounds may be represented by burn wounds such as thermal wounds, chemical wounds and electrical wounds, surgical wounds, stab wounds, and gunshot wounds.

According to further objects of the invention, the material disclosed finds application in the medical, pharmaceutical and cosmetic fields.

### Brief description of the figures

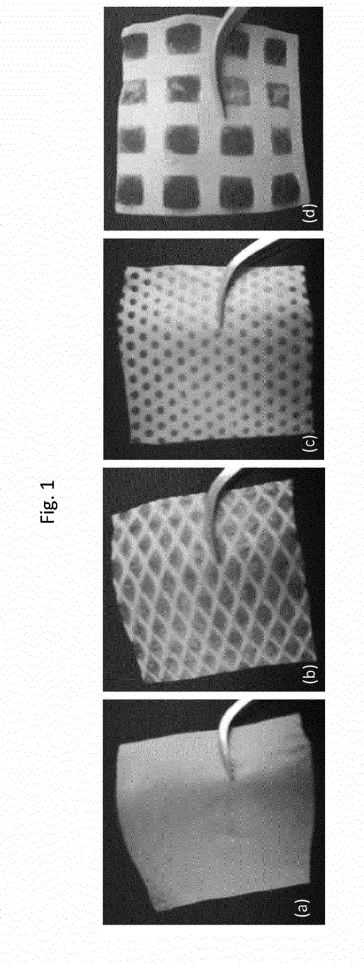

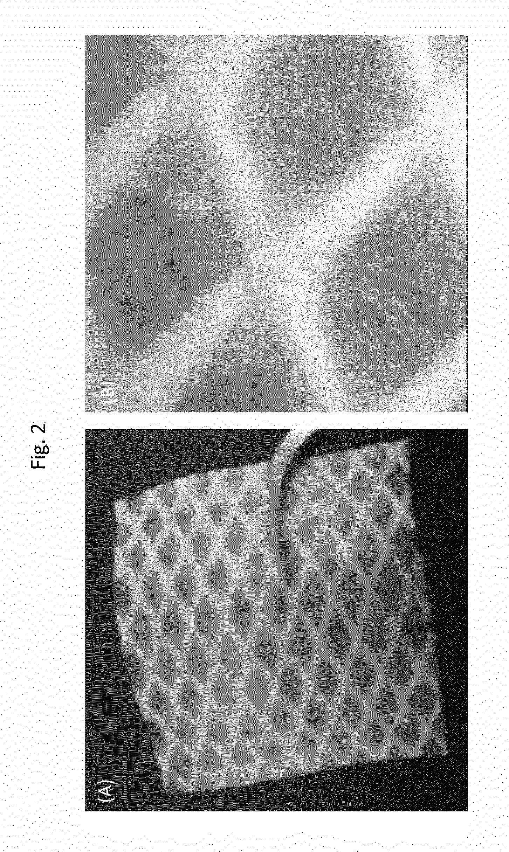

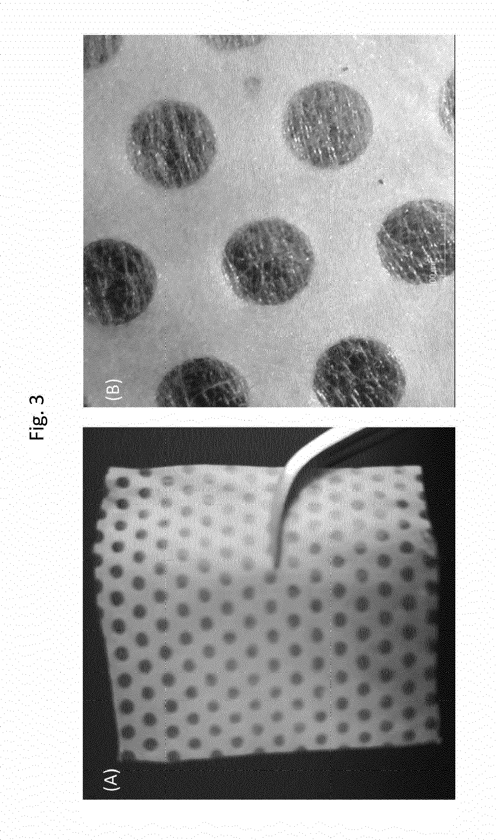

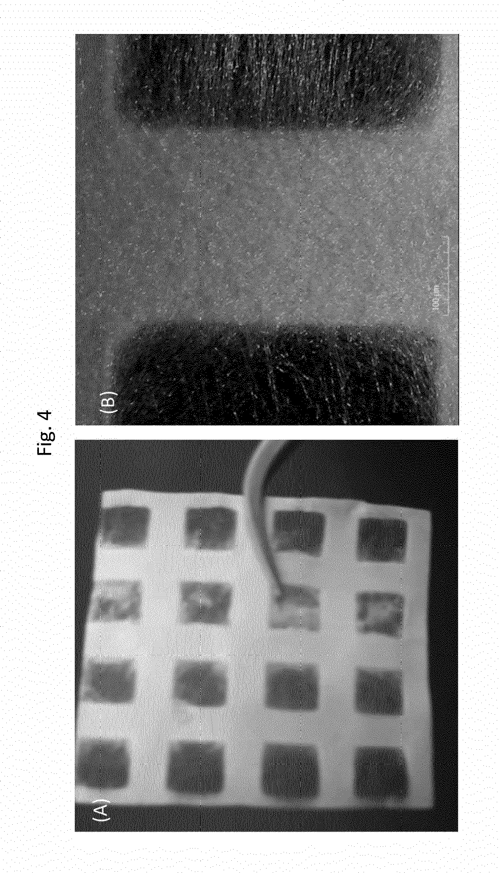

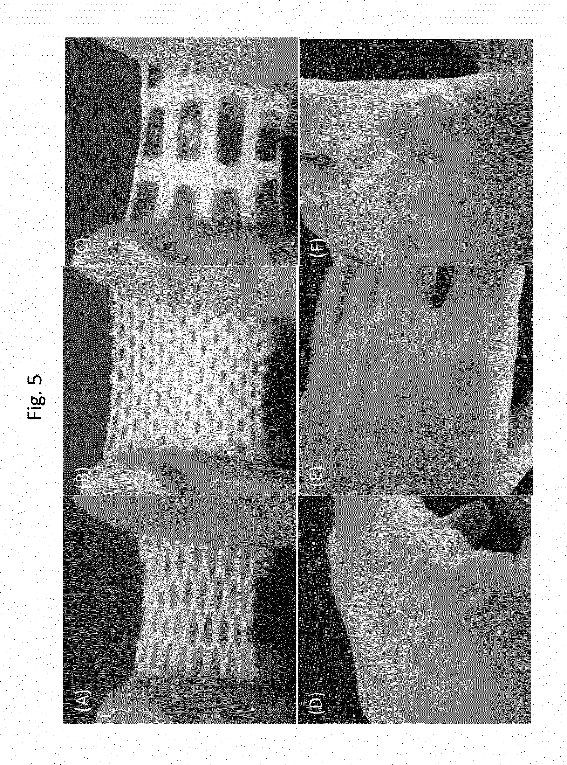

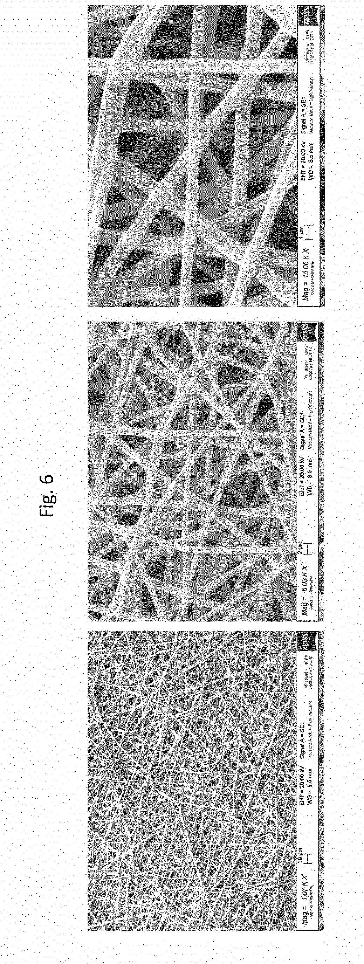

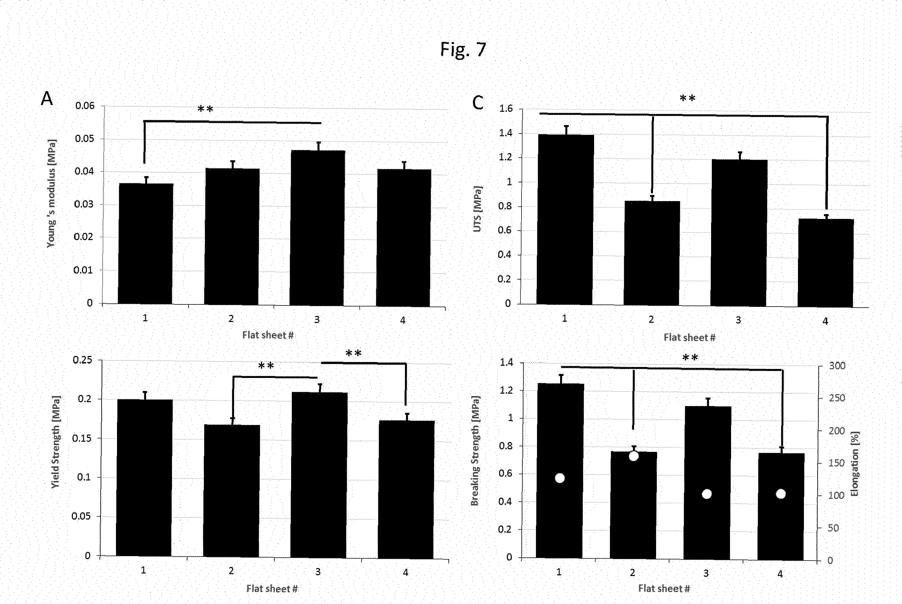

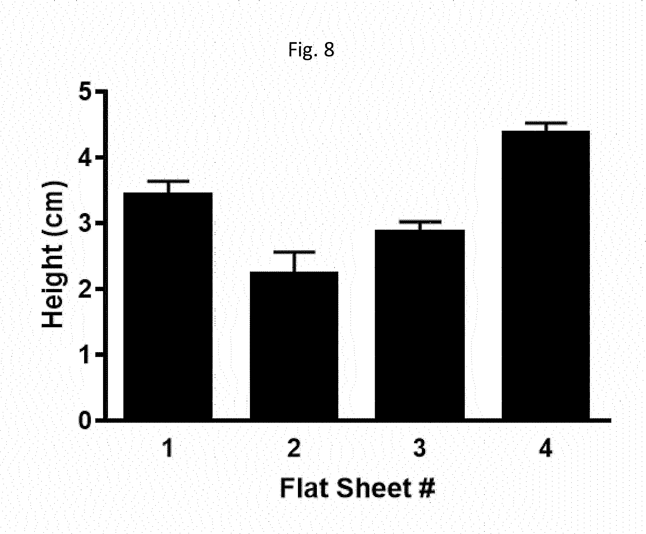

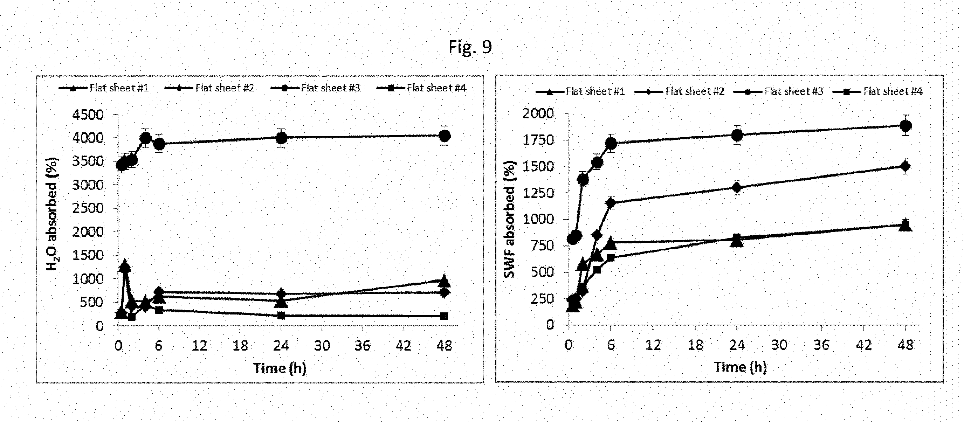

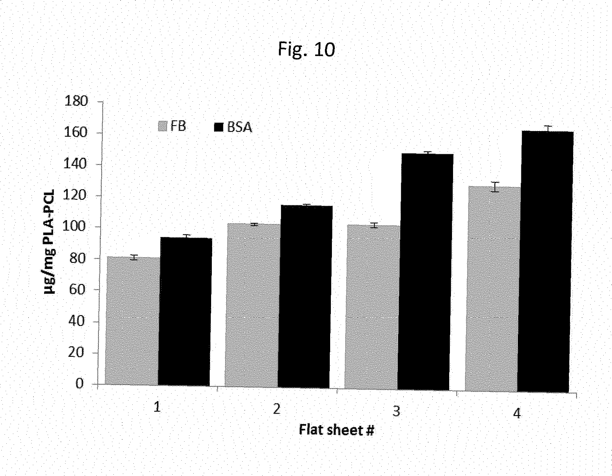

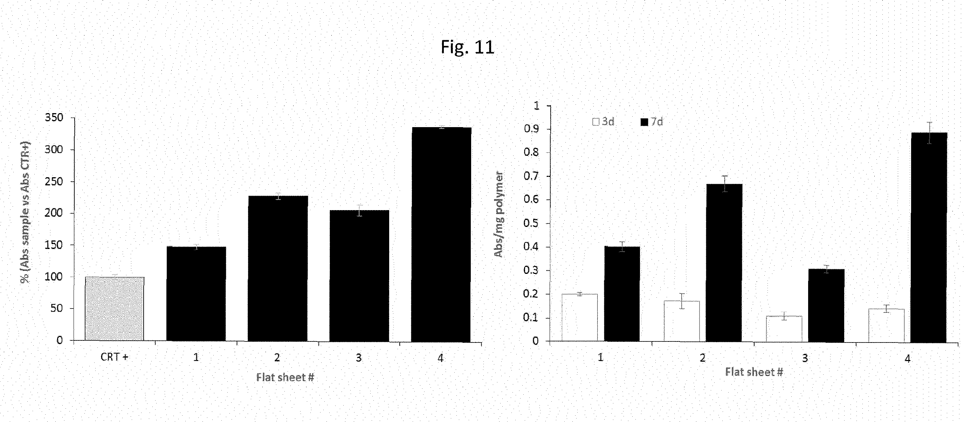

Figure 1 shows images of fiber mesh sheet framework (a) unpatterned, (b) horizontal diamond lattice, (c) circle rhombic lattice and (d) horizontal square lattice; Figure 2A and 2B show image and SEM image of horizontal diamond lattice framework described in Example 3; Figure 3 shows images and SEM image of circle lattice framework described in Example 3; Figure 4 shows image and SEM image of horizontal square lattice framework described in Example 3; Figure 5 reports the images about the stretchability and conformability of 3D pattern fiber platform in Example 2; Figure 6 shows SEM photographs of micro- and nano-fiber platform described in Example 2; Figure 7 shows tensile strength data of micro- and nano-fiber platform described in Example 5; Figure 8 shows wicking behavior of micro- and nano-fiber platform in Example 2; Figure 9 shows results of fluids absorption assay described in Example 2; Figure 10 shows results of protein absorption assay described in Example 6; Figure 11 shows results of MTT assay described in Example 6.

### Detailed description of the invention

According to a first object of the invention, it is disclosed a three-dimensional fiber platform for the preparation of a material.

In particular, said three-dimensional fiber platform comprises a process for preparing a material, which can be referred to as an electrospun membrane, a fiber matrix or an electrospun matrix.

More in particular, the invention process comprises the steps of:
1) preparing a solution of a polymer or of a mixture of polymers,
2) subjecting the solution of a polymer or of a mixture of polymers obtained from step 1) to an electrospinning step.

For the purposes of the present invention, a mixture in step 1) may be prepared with one polymer represented by a biodegradable polymer.

According to one embodiment of the invention, the polymer can be represented by polyester, polysaccharides; or mixtures thereof.

For instance, synthetic polyester can be selected from the group comprising: homopolymers of d,l-lactide, glycolide, caprolactone, p-dioxanon and/or mixtures thereof, copolymer of d,l-lactide and glycolide, or caprolactone, or p-dioxanon, or polyoxyethylene glycols, and/or mixtures thereof, or copolymer of glycolide or lactide and caprolactone, or p-dioxanon, and/or mixtures thereof.

In one embodiment of the invention, the solution of step 1) may comprise a mixture of polymers.

In a preferred embodiment, the solution of step 1) comprises poly-L-lactide-co-poly-caprolactone.

According to one embodiment, the solution of step 1) comprises about 8.0-30% and preferably 15-20% (weight/volume) of polymer or mixture of polymers.

In another embodiment of the invention, the preparation of the solution of step 1) may include the addition of a surfactant.

According to a preferred embodiment, the surfactant is lipophilic.

In a preferred embodiment, the surfactant has a HLB value of about 2-9.

For the purposes of the present invention, a surfactant may be selected in the group comprising the following commercially available products: Brij®, Tetronic, PEG-PPG-PEG Pluronic®.

A preferred surfactant is for instance represented by Polyoxyethylene sorbitan fatty acid esters 80 (having a molecular weight of 1310 Da and a viscosity of 425 mPa·s).

In one embodiment, a surfactant may be added to a final concentration of 0.8-4.8% (volume/volume).

According to the present invention, the step 2) of electrospinning comprises the application of one or a combination of two or more of the following conditions:

| **Condition** | **Value (about)** |
|---|---|
| Voltage | -10 - + 100 kV |
| Flow rate | 0.1-60 ml per hours (1 needle) |
| Distance needle-manifold | 50-150 mm |
| Spinneret width | 0-80 mm |
| Spinneret speed | 0-300 mm/s |
| Electrospinning time | 8-60 min |

According to the embodiment wherein the mixture of PLA-PCL copolymer is used for preparing the solution of step 1), the following conditions may be used:

| **Condition** | **Value (about)** |
|---|---|
| Voltage | -15 - +30 kV |
| Flow rate | 0.1-10 ml per hours (1 needle) |
| Distance needle-manifold | 10-25 cm |
| Spinneret width | 0-80 mm |
| Spinneret speed | 100-200 mm/s |
| Needle cleaning frequency | 1-10 sec |
| Electrospinning time | 8-60 min |

In a preferred embodiment, the following conditions are used when a mixture of PLA-PCL copolymer is used for preparing the solution of step 1):

| **Condition** | **Value (about)** |
|---|---|
| Voltage | +20 kV |
| Flow rate | 0.6 ml per hours (1 needle) |
| Distance needle-manifold | 15 cm |
| Spinneret width | 80 mm |
| Spinneret speed | 100 mm/s |
| Needle cleaning frequency | 1 sec |
| Electrospinning time | 8 min |

According to the present invention, step 2) of electrospinning is performed using a collector and an injection system, wherein the injection system is at a constant distance from the collector.

According to a particular embodiment, step 2) is performed under conditions of controlled relative humidity (RH); preferably, RH should be of between 20-40% and in case nanoparticles are included in the formulation the relative humidity should be less than about 25%.

In a particular embodiment, step 2) may lead to a material having a specific porosity.

The material of the invention is characterized by a porosity, wherein the pore size is about 1-200 µm, preferably of about 10-175 µm and more preferably of about 1-50 µm.

In another particular embodiment, step 2) may lead to an unpatterned material or to a material having a specific texture.

More in particular, said texture may be: horizontal diamond lattice (diamond), circle rhombic lattice (round) or horizontal square lattice (square).

Preferred features of each texture are reported in the following Table 1, reporting the characteristics of area (cm²), pore number and pore area (m²).

| **Texture** | **Area (cm²)** | **Pore number** | **Pore area (m²)** |
|---|---|---|---|
| Diamond | 15 | 680 | 7875.75 |
| Round | 15 | 1548 | 892.76 |
| Square | 15 | 210 | 18098.78 |

In a preferred embodiment of the invention, step 2) of electrospinning results in polymeric fibers having a size of about 0.1-6 µm (microfibers), wherein size is used to refer to the fiber diameter.

In a preferred embodiment of the invention, step 2) of electrospinning results in polymeric fibers having a diameter of about 0.30-15.0 µm, preferably of about 0.30-5.0 µm and even more preferably of 0.30-4.0 µm.

According to a particular embodiment of the invention, the step 2) of electrospinning may result in an amount of nano-sized fibers.

Nano-sized fibers are intended to have a size (diameter) of about 0.02-1 µm.

In one embodiment of the invention, nanofibers may represent about 4.0-75% of the total numbers of fibers.

According to a preferred embodiment, after step 2) it is carried out a step 3), wherein the obtained material is subjected to drying, in order to remove the solvent residuals.

According to another embodiment of the invention, after step 3) the obtained material may be subjected to post-processing step phase 4) for providing the material with further desirable properties.

Said post-processing step 4) may comprise one or more treatment having, for instance, the purpose of: functionalization to attach an active ingredient, functionalization to attach molecules, such as RGD for promoting the cell attachment of the membrane, and/or engineering with cells for skin regeneration.

According to a second object, the present invention discloses a process for the preparation of a material according to the above description loaded with a drug or an active agent.

For said purposes, the process above disclosed comprises a step 1) wherein the polymeric solution is added with a preparation of the drug to be loaded.

According to one embodiment, loading may be carried out with a drug.

In particular, a drug may be selected in the group comprising anti-inflammatory, anti-fibrotic, anti-microbial agents and silver compounds.

More in particular, a drug may be selected in the group comprising: NSAIDs, pirfenidone, vancomycin, gentamycin, neomycin, silver sulfadiazine, silver nitrate and silver nanoparticles.

According to another embodiment of the invention, loading may be carried out with an active agent.

Preferably, said active agent may be represented by a nucleic acid.

In a preferred embodiment, the nucleic acid is represented by siRNA.

A preparation of siRNA according to the present invention may be pre-designed according to the specific needs.

Preparations of siRNA suitable for the present invention are commercially available, such as, for instance from Sigma Aldrich (Mission® customized siRNA).

According to one embodiment of the invention, one or more drugs may be loaded to the system, optionally comprising a nucleic acid.

According to one embodiment of the present invention, the drug or the active agent may be loaded on a carrier.

Preferably, the carrier is in the form of nanoparticles, thereby providing a nanocarrier.

The nanocarrier may have a diameter of about 50-200 nm.

For the purposes of the present invention, the carrier may be represented by chitosan nanoparticles.

In particular, the nanoparticles may be obtained from chitosan or chitosan salts or chitosan derivatives selected from the group comprising, for instance: chitosan hydrochloride, carboxymethylchitosan, chitosan acetate, chitosan glutamate, having different molecular weights, degree of deacetylation and theoretical charge ratio between amino groups of chitosan and phosphate groups of nucleic acids.

A variety of chitosan polymers at medical grade are commercially available.

In particular, the drug may be entrapped within the colloidal matrix of nanoparticle or form a coating on the particle surface via conjugation or adsorption.

According to a preferred embodiment of the invention, when the active agent is siRNA, a nanocarrier is represented by chitosan nanoparticles.

For said purposes, a suspension of chitosan or chitosan derivative nanoparticles is prepared and added with the required drug or active agent, like siRNA to obtain loaded chitosan nanoparticles.

Accordingly, step 1) of the process comprises the addition to the solution of a polymer or of a mixture of polymers of the carrier onto which the drug or the active agent has been loaded.

In a preferred embodiment of the invention, from step 1) of the process it is obtained a suspension of chitosan nanoparticles containing nucleic acid as the loaded carrier in PLA-PCL copolymer.

For the purposes of the present invention, it can be used a chitosan/PLA-PCL ratio from about 1:99 to about 20:80 (weight/weight) and preferably of about 3:97 to 10:90.

The material produced according to the method of the invention is intended to be sterile and it may be produced by an isolator technology or in Restricted Access Barrier System (RABS), and the associated processes, may be designed so as to provide maximum protection of the grade A.

In order to grant aseptic conditions inside the machine process area, the isolator may be designed as highly automated system (following Annex 1 Manufacture of Sterile Medicinal Products).

According to one embodiment of the present invention, the material may be obtained in the form of a delivery platform.

In the following table there are reported the preferred features of a material obtained according to the present invention as a delivery platform, which has one of the particular lattice frameworks:

| **Flat sheet #** | **Lattice Framework** | **Fiber mean diameter (µm)** | **Fiber diameter range (µm)** | **Nano-ranged fiber (%)** | **Porosity (%)** |
|---|---|---|---|---|---|
| **1** | **Unpattern** | 5.0 ± 1.7 | 0.33 - 14.7 | 4.30 | 61.60 |
| **2** | **Horizontal diamond lattice** | 2.68 ± 0.63 | 0.32 - 4.20 | 65.1 | 55.60 |
| **3** | **Circle rhombic lattice** | 4.45 ± 2.08 | 0.30 - 5.22 | 19.1 | 56.70 |
| **4** | **Horizontal square lattice** | 2.16 ± 0.80 | 0.33 - 4.24 | 76.0 | 55.4 |

Example of unpattern (3D fiber flat sheet) and 3D patterned flat sheets having horizontal diamond lattice, circle rhombic lattice, and horizontal square lattice are shown in Figures 1 (a-c), Figures 2 (a) and (b) (horizontal diamond lattice), 3 (circle rhombic lattice) and 4 (horizontal square lattice).

In some embodiments, the lattice framework is substantially uniform as shown in Figures 2A, B and Figures 3A, B, Figures 4A, B.

In certain embodiments, the lattice framework based on fiber forming a well interconnected structure may serve to make the 3D Fiber Delivery Platform stretchable and conformable by allowing the flat sheet to be bent and curved without kinking as shown in Figures 5A-B-C.

In some embodiments, the interconnected polymeric electrospun fibers which form the flat sheet may have a porous architecture, which may mimic the extracellular matrix (ECM) of tissues surrounding the site of application.

The well interconnected and high porous structure may permit cell to grow inducing tissue regeneration and leading to re-epithelialization at the interface between the fiber platform and the tissue surrounding the wound bed.

Example of 3D fiber highly porous and interconnected structure are illustrated in Figure 6.

In particular, Figure 6 shows SEM micrographs of electrospun fiber section and interconnected network formed during the deposition of fibers on the collector.

The section has not submitted to further treatment after production.

According to another embodiment, the material produced according to the present invention may be obtained in the form of a dressing.

In particular, the material is characterized by tensile properties rendering it stretchable and conformable (Figure 5A-F).

Tensile properties of the fiber dressing may be measured by Mark 10 tensile tester (ESM 303 Instrument) equipped with a clip-on extensometer to measure specimen extension.

The employed cross-head speed may be 0.5 mm min-1, and the maximum force recorded may be used to obtain the ultimate tensile strength, UTS [MPa].

As for the Young's module the material according to the invention has a value of 0.03-0.05 MPa.

As for the yield strength, the material of the invention is characterized by a yield strength of about 0.15-0.2 MPa.

As for the ultimate tensile strength, the material of the invention is characterized by a value of 0.7-1.4 MPa.

As for the breaking strength it is preferably less that about 1.2 MPa.

As for the elongation percentage, it is preferably higher than about 120%.

In a preferred embodiment, the fiber dressing may have a vertical wicking (which measures the capability of the membrane to uptake and retain exudate (the term exudate being intended to identify liquid produced from wounds, having preferably a high content of protein and having a specific gravity higher than 1.020) or physiological fluids) capability of about 2-5 cm.

In a preferred embodiment, the fiber dressing may have an absorption capability versus time of about 2000 - 7000%

In a preferred embodiment, the fiber dressing has a fluid absorption of about 3500-4000%.

In another preferred embodiment, the fiber dressing has a fluid absorption of less than about 1800%.

The lattice framework forms a well interconnected structure, which renders the dressing stretchable and conformable by allowing the flat sheet to be stretched as illustrated in Figures 5A-C.

According to another embodiment of the present invention, the material may be obtained in the form of a three dimensional scaffold.

In particular, the material is characterized by hemocompatibility and plasma protein adsorption.

Hemocompatibility is measured through the adsorption of bovine serum albumin (BSA) and fibrinogen (FB) through the Bicinconinic Assay (BA) at the wavelength of 562 nm.

Plasma protein adsorption may be of about 100-200 mg/ per mg of PLA-PCL.

The protein adsorption percentage may be of about 100-200% (calculated with respect to mg of polymer membrane).

In a third object, the present invention discloses a fiber delivery platform, a fiber dressing or a fiber scaffold, optionally loaded with a drug or an active agent, for use as a medicament.

Particularly, it is disclosed the use as a medicament for the treatment and healing of wounds.

More in particular, wounds may be selected in the group comprising: burn wounds such as thermal wounds, chemical wounds and electrical wounds, surgical wounds, stab wounds, gunshot wounds.

According to further objects of the invention, the material disclosed finds application in the medical, pharmaceutical and cosmetic fields.

In particular, the material, both drug loaded and unloaded, may find application for wound dressing, burn dressing, tissue regeneration, as medicated dressing, cosmetic mask, cosmetic wrap dressing, anti-adhesion physical barrier and drug carrier.

Therefore, a wound dressing, a burn dressing, a medicated dressing, a cosmetic mask, a cosmetic wrap dressing, an anti-adhesion physical barrier and a drug carrier comprising the material of the present description are further object of the invention.

The present invention will be further described by the following non-limiting examples.

### EXAMPLE 1

### Preparation of PLA-PCL electrospun fiber mesh sheet

Poly-L-lactide-co-poly-caprolactone, PLA-PCL (2g, Resomers LC 703, molar ratio 70:30, molecular weight 160 kDa, glass transition temperature 37°C, intrinsic viscosity between 1.3-1.8 dL/G, Evonik Nutrition & Care, Darmstadt, Germany) has been solved in dichloro methane (DCM, 13.3 mL) and maintained under slow magnetic stirring (100 rpm)(MULTISTIRRER, magnetic stirrer VELP scientific) for three hours in a water-ice bath.

All electrospinning runs have been performed by an electrospinning GMP oriented industrial nanofiber production machine (Nanon-01A, MEEC Instruments, Ltd., Ogori-shi, Fukuoka, Japan) at room temperature and keeping RH at 25%.

The PLA-PCL solution was loaded into a plastic syringe w/o stopper (5 ml Syringe, Luer-Lok™ Tip, laagstraat 57,B-9140 Temse, Belgium) and injected through a 18G needle at specific flow rates, spinneret speed and spinneret width. The distance between the needle and the manifold was maintained constant at 15 cm. The voltage has been set at +20 kV and flow rate at 0.6 mL/h; spinneret width and speed were 80 mm and 100 mm/sec, respectively. All sheet mesh sheets have been electrospun for 10 minutes and collected on flat collector (14 cm x 25 cm).

3D patterned nanofiber mesh sheets having different textures were prepared using conductive plates placed on collector and having different textures.

Nanofiber mesh sheets were detached from conductive plates, weighed and stored in dryer for the following 48 hours to remove any traces of solvent. The samples were subsequently stored at 4 + 1°C and controlled humidity (33 ± 1%).

### EXAMPLE 2

### Preparation of electrospun nanofiber mesh sheet containing siRNA/drug loaded nanocarrier

Electrospun nanofiber mesh sheet containing drug loaded nanocarrier were prepared incorporating siRNA/drug nanocarrier (16 mg) in PLA-PCL polymer solution (704 mg), PLA-PCL and nanocarrier weight ratio has been set up at 1:44 w/w.

Nanocarrier was recovered by centrifuge (Centrifuge 5417 Eppendorf) in compatible microtubes (Snap-Cap Microcentrifuge Biopur™ Safe-Lock™ Tube Eppendorf); the pellet was resuspended in DCM supplemented with surfactant (Span 80, 4.8 v/v%) and vortexed (Advanced Vortex Mixer VELP scientifica) for one minute. Each suspension has been characterized in terms of particle size and size distribution (NICOMP 380 ZLS, Particle sizing system, CA, USA) before electrospinning.

The drug loaded nanocarrier PLA-PCL suspension was loaded into a plastic syringe w/o stopper and injected through a 18G needle at flow rate at 0.6 mL/h; spinneret width and speed were 80 mm and 100 mm/sec, respectively. All sheet mesh sheets have been electrospun for 10 minutes and collected on flat collector (14 cm x 25 cm) maintaining the distance between the needle and the manifold at 15 cm. The voltage has been set at +20 kV. NPs loaded electrospun nanofiber mesh sheets have been prepared at 24 ± 5°C and RH was increased at 30%. Electrospun mesh sheets were detached by conductive plate and stored at 4 ± 1°C, controlled humidity (33 ± 1%) until further characterization.

### EXAMPLE 3

### Morphological characterization of fiber mesh sheet

Both light microscope (OPTIKA B-293 Trinocular), and scanning electron microscopy (SEM, Zeiss EVO MA10 Carl Zeiss, Oberkochen Germany) imaging were used. Each SEM sample has been attached using carbon coated tape to a SEM sample stub, and following coated with gold in argon atmosphere. The images were acquired at high voltage (+20kV), in high vacuum, at temperature environment and at different magnifications 500 X, 1.02 kX, 5.04 kX, 10 kX and 15 kX. Dimensional analysis of nanofibers (fiber diameter, fibers size distribution, pore distribution and fiber orientation) was carried out by using Image J.

### EXAMPLE 4

### 3D Fiber Delivery Platform

Figures 6 shows SEM micrographs of electrospun fiber section and interconnected network formed during the deposition of fibers on the collector. The interconnected electrospun fiber network is made from poly (L-lactide)-co-po|y(ε-caprolactone) block copolymer having weight ratio of poly (L-lactide) to po|y(ε-caprolactone) of about 70:30 (referred to herein as PLA-PCL 70:30), average molecular weight 160 kDa and intrinsic viscosity ranged from 1.3 to 1.8 dL/g. The PLA-PCL polymer is dissolved in DCM solvent (15% w/v) maintaining under stirring in water/ice bath; the polymer solution is sonicated to remove air bubble and loaded in a plastic syringe (Luer-LokTM). The electrospinning parameters include +20 kV, 6 mL/h flow rate, 15 cm tip-to-collector distance, 80 mm spinneret width, 100 mm/sec spinneret speed and cleaning frequency every 30 sec. The selected electrospinning parameters provide bead-free fibers and increased stiffness of the polymeric electrospun fiber depending on spinning time.

### EXAMPLE 5

### 3D Fiber Wound Dressing

A section of the material is produced according to Example 1.

Specimens having dog-bone shape may be cutting die (Model 10019091) manufactured to produce a bar specimens measuring 80±0.7x10±0.4x4±0.3 mm following ASTM D 882. The certification of cutting die meets ISO 17025:2017. Tensile properties of the fiber dressing may be measured by Mark 10 tensile tester (ESM 303 Instrument) equipped with a clip-on extensometer to measure specimen extension. The employed cross-head speed may be 0.5 mm min-1, and the maximum force recorded may be used to obtain the ultimate tensile strength, UTS [MPa]. Figures 7 is a plot of the tensile data for fiber electrospun flat sheet made from PLA-PCL 70:30. The plot A in Figures 7 charts Young's modulus values of fiber dressings.
The plot C in Figures 7 charts ultimate tensile strength of fiber dressings.

Figures 8 is a plot of the vertical wicking data for fiber electrospun flat sheet made from PLA-PCL 70:30.

Figures 9 is a plot of absorption capability (percentage values of water and SWF absorbed) of fiber dressing versus time. The specimens may be incubated in water or in SWF for 48 hours in incubator at 34°C (skin temperature); the amount of fluid handled by the specimen may be calculated gravimetrically after removing the excess of fluid. In some embodiments, the fiber dressing may absorb the maximum of fluid (water or SWF) after six hours of incubation.

### EXAMPLE 6

### 3D Fiber Scaffold

Figures 10 is a plot of the hemocompatibility data for fiber electrospun flat sheet made from PLA-PCL 70:30.

The attachment and cell proliferation may be evaluated through MTT Assay (3-(4,5-dimethylthiazol-2-yl)-2,5-diphenyltetrazolium bromide). The specimens may be fixed on 12-well cell crown and hydrated in sterile cell culture media (DMEM, pH 7.4). After hydration, cell suspension (100 kcell/specimen) may be seeded on the surface of specimen and maintained at room temperature in sterile condition for 15 min, to boost the attachment of cells on fiber surface. The specimens may be incubated in cell culture media at 37°C, 5% CO₂ for scheduled time. The optical density (OD) may be measured at a wavelength of 570 nm and at a reference wavelength of 670 nm. The cell viability may be expressed as percentage (%) of the DOs/DOc ratio, where DOs is the mean value of the optical density of the samples object of the test, and DOc is the average value of untreated cells. Figures 11 is a plot of the cell attachment and proliferation data for fiber electrospun flat sheet made from PLA-PCL 70:30. In all embodiments, the cell culture results in cell attachment and following cell proliferation. The results show that the cell attachment and proliferation may be influenced by the lattice framework, the most promising results may be observed for flat sheet having horizontal diamond lattice and horizontal square lattice, respectively.

From the above disclosure, the several advantages of the present invention will be evident to the person skilled in the art.

As a first advantage, it can be noticed that the material disclosed by the present invention is completely biocompatible and completely biodegradable.

In addition, the material offered by the present patent application has surprisingly shown to maintain the stability and the transfection efficiency of the load drug or active agent.

Even more surprisingly, it has demonstrated to maintain the stability of the nucleic acid comprised therein, thereby representing a promising tool for gene therapy.

Said application is particularly, advantageous, since the developed material is capable of neutralizing siRNA negative charge to allow *in vivo* permeation of siRNA, while overcoming the shortcoming of a rapidly reduced treatment efficiency due to siRNA low stability and easy *in vivo* degradation.

The material disclosed has also demonstrated to tune its absorbent performances and to manage the wound exudate, thereby representing a promising tool for the wound healing.

Further, the material of the invention has shown to support and promote the cell attachment and proliferation, thereby representing a promising tool for tissue regeneration.

Moreover, the material can be adapted or integrated in the automatized production of sterile pharmaceutical and biotechnological products, in particular for the advanced therapy medicinal products (ATMPs), thereby avoiding and overcoming the criticalities connected with the sterilization process of the materials, as already known in the art.

As it is above disclosed, the material of the invention may be obtained as a delivery platform, as a wound dressing and as a fiber scaffold.

Each form may then find more specific but not limiting applications in view of the peculiar properties of the material.

## Claims

1. A process for the preparation of a polymeric material, comprising the steps of:
1) preparing a solution of a polymer or of a mixture of polymers,
2) subjecting the solution of a polymer or of a mixture of polymers obtained from step 1) to an electrospinning step, wherein in step 1) the polymer is selected between polyester and polysaccharides, wherein the polyester is selected from the group comprising: homopolymers of d,l-lactide, glycolide, caprolactone, p-dioxanon and/or mixtures thereof, copolymer of d,l-lactide and glycolide, or caprolactone, or p-dioxanon, or polyoxyethylene glycols, and/or mixtures thereof, or copolymer of glycolide or lactide and caprolactone, or p-dioxanon, and/or mixtures thereof.

2. The process according to claim 1, wherein in step 1) it is added a surfactant to a final concentration of 0.8-4.8% (volume/volume).

3. The process according to any one of the preceding claims, wherein the solution of step 1) comprises about 8.0-30% (weight/volume) of polymer or mixture of polymers.

4. The process according to any one of the preceding claims, wherein the solution of step 1) comprises poly-L-lactide-co-poly-caprolactone.

5. The process according to any one of the preceding claims, wherein the solution of step 1) includes a drug or an active agent, which optionally can be loaded onto a nanocarrier.

6. The process according to the preceding claim 5, wherein the solution of step 1) includes a drug or an active agent loaded onto chitosan nanoparticles.

7. The process according to any one of the preceding claims, wherein step 2) is performed under one or a combination of two or more of the following conditions:
| **Condition** | **Value (about)** |
|---|---|
| Voltage | -10 - + 100 kV |
| Flow rate | 0.1-60 ml per hours (1 needle) |
| Distance needle-manifold | 50-150 mm |
| Spinneret width | 0-80 mm |
| Spinneret speed | 0-300 mm/s |
| Electrospinning time | 8-60 min |

8. The material obtained according to the process of any one of the preceding claims 1 to 7.

9. The material according to the preceding claim in the form of a fiber delivery platform, a fiber dressing or a fiber scaffold.

10. The material according to the preceding claim 8 or 9, having a fiber size of about 0.1-6 µm, preferably of about 0.30-15.0 µm, more preferably of about 0.30-5.0 µm and even more preferably of about 0.30-4.0 µm, and optionally nanofiber in an amount of about 4.0-75% of the total numbers of fibers having a size of about 0.02-1 µm.

11. The material according to any one of the preceding claims 8 to 10, having one or more of the following properties:
| | |
|---|---|
| Young's Module | 0.03-0.05 MPa |
| Yield Strength | 0.15-0.2 MPa |
| Ultimate Tensile Strength | 0.7-1.4 MPa |
| Breaking Strength | 1.2 MPa |
| Elongation Percentage | >120% |
| Vertical Wicking | 2-5 cm |

12. The material according to any one of the preceding claims from 8 to 11 as a medicament.

13. The material according to any one of the preceding claims from 8 to 11 according to the preceding claim as a medicament for the treatment and healing of wounds.

14. The material obtained according to any one of the preceding claims 12 or 13 as a medicament for the treatment and healing of wounds, wherein said wounds is selected from the group comprising: burn wounds such as thermal wounds, chemical wounds and electrical wounds, surgical wounds, stab wounds, gunshot wounds.

15. A wound dressing, a burn dressing, a medicated dressing, a cosmetic mask, a cosmetic wrap dressing, an anti-adhesion physical barrier and a drug carrier comprising the material according to any one of the preceding claims from 8 to 11.
